(19) Europäisches Patentamt
European Patent Office
Office européen des brevets

(11) Veröffentlichungsnummer : **0 141 317 B1**

(12) **EUROPÄISCHE PATENTSCHRIFT**

(45) Veröffentlichungstag der Patentschrift : **20.01.88**

(21) Anmeldenummer : **84112283.1**

(22) Anmeldetag : **12.10.84**

(51) Int. Cl.[4] : **C 07 D487/04**, A 01 N 43/90 // (C07D487/04, 249:00, 239:00),(C07D487/04, 239:00, 231:00)

(54) **7-Amino-azolo[1,5-a]-pyrimidine und diese enthaltende Fungizide.**

(30) Priorität : **21.10.83 DE 3338292**

(43) Veröffentlichungstag der Anmeldung : **15.05.85 Patentblatt 85/20**

(45) Bekanntmachung des Hinweises auf die Patenterteilung : **20.01.88 Patentblatt 88/03**

(84) Benannte Vertragsstaaten : **AT BE CH DE FR GB IT LI NL SE**

(56) Entgegenhaltungen :
**EP-A- 0 071 792**
**CHEMICAL ABSTRACTS, Band 68, 1968, Seite 9252, no. 95842s, Columbus, Ohio, US; & JP - A - 11 751 (SHIONOGI & CO., LTD.) 04-07-1964**
**CHEMICAL ABSTRACTS, Band 60, 1964, Nr. 523d, Columbus, Ohio, US; YA.A. LEVIN u.a.: "Condensation of 3-amino-1,2,4-triazoles with diaceto- and dipropionitriles", ZH. OBSHCH. KHIM. 33(8), 2673-7(1963)**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Eicken, Karl, Dr.**
**Am Huettenwingert 12**
**D-6706 Wachenheim (DE)**
Erfinder : **Graf, Hermann, Dr.**
**Ginsterstrasse 15**
**D-6704 Mutterstadt (DE)**
Erfinder : **Gramlich, Walter, Dr.**
**Auf der Höhe 11**
**D-6803 Edingen-Neckarhausen (DE)**
Erfinder : **Sauter, Hubert, Dr.**
**Neckarpromenade**
**D-6800 Mannheim 1 (DE)**
Erfinder : **Rentzea, Costin, Dr.**
**Richard-Kuhn-Strasse 1-3**
**D-6900 Heidelberg (DE)**
Erfinder : **Pommer, Ernst-Heinrich, Dr.**
**Berliner Platz 7**
**D-6703 Limburgerhof (DE)**
Erfinder : **Ammermann, Eberhard, Dr.**
**Sachsenstrasse 3**
**D-6700 Ludwigshafen (DE)**

EP 0 141 317 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft 7-Amino-azolo [1,5-a]-pyrimidine, Verfahren zu ihrer Herstellung und diese enthaltende Fungizide.

Es ist bekannt, daß 7-Amino-azolo [1,5-a]-pyrimidine pharmakologische Eigenschaften besitzen (FR-PS 2 448 542 ; DD-PS 99 794 ; DD-PS 55 956 ; J. pharm. Soc. Japan 84 (1964), S. 1113 bis 1118).

Es ist ferner bekannt, 7-Amino-azolo [1,5-a]-pyrimidine, die in 6-Stellung einen Phenylrest tragen, als Fungizide zu verwenden (EP-A-0 071 792). Auch die Verbindung 7-Amino-6-hexyl-5-methyl-[1,2,4]-triazolo [1,5-a]-pyrimidin ist bekannt (Chemical Abstracts, Band 60, 1964, Nr. 523 d).

Es wurde nun gefunden, daß 7-Amino-azolo [1,5-a]-pyrimidine der Formel

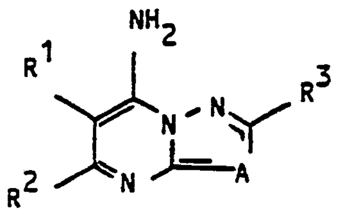

(I)

worin

$R^1$ gegebenenfalls durch $C_1$-$C_{18}$-Alkoxi oder Halogen substituiertes $C_4$-$C_{18}$-Alkyl

$R^2$ und $R^3$ Wasserstoff oder $C_1$-$C_4$-Alkyl,

A ein Stickstoffatom oder eine $CR^4$-Gruppe bedeutet, wobei

$R^4$ die Bedeutung Wasserstoff, $C_1$-$C_4$-Alkyl oder Halogen hat,

eine gute fungizide Wirkung, insbesondere gegen Phycomyceten, haben.

Unter den Resten $R^1$ sind beispielsweise gegebenfalls durch Fluor, Chlor, Brom oder $C_1$- bis $C_{18}$-Alkoxi substituiertes $C_4$- bis $C_{18}$-Alkyl, z. B. $C_4$-Alkyl, $C_5$-Alkyl, $C_6$-Alkyl, $C_8$-Alkyl zu verstehen.

Unter den Resten $R^2$, $R^3$ und $R^4$ ist beispielsweise Wasserstoff oder $C_1$- bis $C_4$-Alkyl (z. B. Methyl) zu verstehen. Darüber hinaus kann $R^4$ beispielsweise Chlor oder Brom bedeuten.

Unter Alkyl oder Alkyl einer Alkoxigruppe ist je nach Zahl der angegebenen Kohlenstoffatomen Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl oder Octadecyl und ihre Isomeren zu verstehen.

Es wurde ferner gefunden, daß man 7-Amino-azolo [1,5-a]-pyrimidine der Formel I erhält, indem man einen substituierten β-Ketoester der Formel II

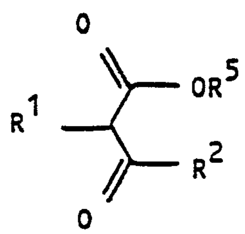

(II)

in der $R^1$ und $R^2$ die oben genannten Bedeutungen haben und $R^5$ für einen niederen Alkylrest steht, mit einem Aminoazol der Formel III

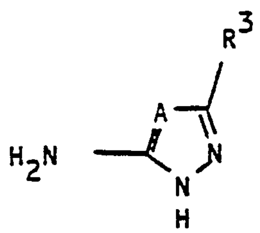

(III)

in welcher A, $R^3$ und $R^4$ die oben angegebenen Bedeutungen haben, zu Kondensationsprodukten der Formel V

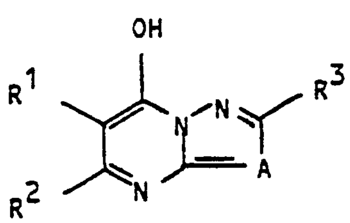

(V)

in welcher $R^1$, $R^2$, $R^3$ und A die oben angegebenen Bedeutungen haben, umsetzt und diese Kondensationsprodukte der Formel V nach Halogenierung der Hydroxigruppe mit Ammoniak umsetzt.

Die β-Ketoester können hergestellt werden wie in Organic Synthesis Coll. Vol. 1, S. 248 beschrieben.

Die Umsetzung der substituierten β-Ketoester der Formel II mit den Aminoazolen der Formel III kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, Amide wie Dimethylformamid, Diethylformamid, Dibutylformamid, N,N-Dimethylacetamid, niedere Alkansäuren wie Ameisensäure, Essigsäure, Propionsäure und Mischungen dieser Lösungsmittel mit Wasser in Frage. Die Umsetzungstemperaturen liegen zwischen 50 und 300 °C, vorzugsweise bei 50 bis 150 °C, wenn in Lösung gearbeitet wird.

Die so erhaltenen Kondensationsprodukte der Formel V fallen aus den Reaktionslösungen meist in reiner Form aus und werden nach dem Waschen mit dem gleichen Lösungsmittel oder mit Wasser und anschließendem Trocknen mit Halogenierungsmitteln, insbesondere Chlorierungsmitteln, wie Phosphorchloriden, vorzugsweise Phosphoroxitrichlorid, bei 50 °C bis 150 °C vorzugsweise in überschüssigem Phosphoroxitrichlorid bei Rückflußtemperatur chloriert. Nach dem Verdampfen des überschüssigen Phosphoroxitrichlorids wird der Rückstand mit Eiswasser gegebenenfalls unter Zusatz eines mit Wasser nicht mischbaren Lösungsmittels behandelt. Das aus der getrockneten organischen Phase gegebenenfalls nach Verdampfung des inerten Lösungsmittels isolierte Chlorierungsprodukt ist meist sehr rein und wird anschließend mit Ammoniak in inerten Lösungsmitteln bei 100 °C bis 200 °C zu den neuen 7-Amino-azolo [1,5-a]-pyrimidinen umgesetzt. Die Reaktion wird vorzugsweise mit 1- bis 10-molarem Überschuß an Ammoniak unter Druck von 1 bis 100 bar durchgeführt.

Die neuen 7-Amino-azolo [1,5-a]-pyrimidine werden gegebenenfalls nach Verdampfen des Lösungsmittels durch Anteigen mit Wasser als kristalline, meist sehr reine Verbindungen isoliert.

Es wurde ferner gefunden, daß man 7-Amino-azolo [1,5-a]-pyrimidine der Formel I erhält, indem man substituierte Alkylcyanide der Formel

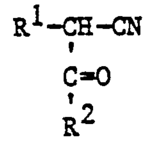

(VI)

in welcher $R^1$ und $R^2$ die oben angegebenen Bedeutungen haben, mit Aminoazolen der Formel III,

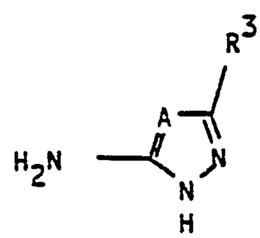

(III)

in welcher A, $R^3$ und $R^4$ die oben angegebenen Bedeutungen haben, umsetzt.

Die Umsetzung kann in Gegenwart oder Abwesenheit von Lösungsmitteln durchgeführt werden. Vorteilhaft ist es, solche Lösungsmittel zu verwenden, gegenüber denen die Einsatzstoffe weitgehend inert sind und in denen sie ganz oder teilweise löslich sind. Als Lösungsmittel kommen insbesondere Alkohole wie Ethanol, Propanole, Butanole, Glykole oder Glykolmonoether, Diethylenglykole oder deren Monoether, Amide wie Dimethylformamid, Diethylformamid, Dibutylformamid, N,N-Dimethylacetamid, niedere Alkansäuren wie Ameisensäure, Essigsäure, Propionsäure und Mischungen dieser Lösungsmittel mit Wasser in Frage. Die Umsetzungstemperaturen liegen zwischen 50 und 300 °C, vorzugsweise bei 50 bis 150 °C, wenn in Lösung gearbeitet wird.

Die neuen 7-Amino-azolo [1,5-a]-pyrimidine werden gegebenenfalls nach Verdampfen des Lösungsmittels oder Verdünnen mit Wasser als kristalline, meist sehr reine Verbindungen isoliert. Bei Verwendung von niederen Alkansäuren als Lösungsmittel ist es zweckmäßig, gegebenenfalls nach teilweisem Verdampfen der Alkansäure, die Reste der Alkansäure durch Zugabe von wäßrigem Alkali zu neutralisieren, wobei die neuen 7-Amino-azolo [1,5-a]-pyrimidine meist in sehr reiner Form auskristallisieren.

Die für die Herstellung der 7-Amino-azolo [1,5-a]-pyrimidine benötigten substituierten Alkylcyanide der Formel

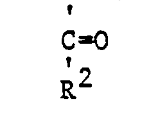

sind teilweise bekannt oder können nach bekannten Methoden aus Alkylcyaniden und Carbonsäureestern mit starken Basen, z. B. Alkalihydriden, Alkaliamiden oder Metallalkylen, hergestellt werden (J. Amer. Chem. Soc. 73, (1951), S. 3766).

Allgemeine Herstellungsvorschriften für die substituierten Alkylcyanide der Formel VI

1,5 Mol Natriumhydrid wird in 1 l Toluol eingetragen und anschließend 1,0 Mol eines Alkylcyanids und dann 2,0 Mol eines Carbonsäureesters unter Rühren zugetropft, wobei die Temperatur auf 40 bis 50 °C ansteigt. Nach 2 stündigem Nachrühren bei 75 bis 80 °C wird abgekühlt und mit 2 l Wasser versetzt. Aus der wäßrigen Phase isoliert man nach zweimaligem Waschen mit 0,2 l Toluol durch Ansäuern mit halbkonzentrierter (etwa 50 Gew.%) Schwefelsäure auf pH 2 das substituierte Alkylcyanid der Formel VI.

## Beispiele

1. Herstellung von 7-Amino-2,5-dimethyl-6-n-octyl-pyrazolo [1,5-a]-pyrimidin (Verbindung 2)

a) 7-Hydroxi-2,5-dimethyl-6-n-octylpyrazolo [1,5-a]-pyrimidin 200 g 2-n-Octylacetessigsäuremethylester und 94 g 3(5)-Amino-5(3)-methylpyrazol werden in 400 ml n-Butanol 5 Stunden am Rückfluß erhitzt. Nach dem Abkühlen, Absaugen, Waschen mit kaltem Methanol und Trocknen im Vakuum bei 60 °C erhält man 191 g 7-Hydroxi-2,5-dimethyl-6-n-octylpyrazolo [1,5-a]-pyrimidin vom Fp. 199 °C.

b) 7-Chlor-2,5-dimethyl-6-n-octylpyrazolo [1,5-a]-pyrimidin 190 g des Kondensationsproduktes aus a) werden in 550 ml Phosphoroxitrichlorid 1,5 Stunden am Rückfluß erhitzt. Nach dem Verdampfen des überschüssigen Phosphoroxitrichlorids im Vakuum wird der Rückstand mit 500 ml $CH_2Cl_2$ und 500 ml Eiswasser verrührt. Die organische Phase wird abgetrennt und dreimal mit je 100 ml Eiswasser gewaschen und über Natriumsulfat getrocknet. Nach dem Filtrieren und Verdampfen des Lösungsmittels im Vakuum isoliert man 179 g 7-Chlor-2,5-dimethyl-6-n-octyl-pyrazolo [1,5-a]-pyrimidin als zähe Masse.

c) 7-Amino-2,5-dimethyl-6-n-octylpyrazolo [1,5-a]-pyrimidin 179 g des Chlorids aus b) werden mit 1 300 ml Ethanol in einen 2,5 l Autoklav gegeben, 85 g Ammoniak aufgepreßt und 8 Stunden bei 150 °C bei 30 bar gerührt. Aus dem Reaktionsaustrag isoliert man nach Aufkonzentrieren im Vakuum, Anteigen mit 1 000 ml Wasser Absaugen und Trocknen im Vakuum bei 70 °C 133 g 7-Amino-2,5-dimethyl-6-n-octyl-pyrazolo [1,5-a]-pyrimidin vom Fp. 169 °C (Verbindung 2).

2. Herstellung von 7-Amino-6-n-butyl-5-methyl-[1,2,4]-triazolo [1,5-a]-pyrimidin (Verbindung 29)

a) 2-Acetyl-n-capronsäure-nitril

In 500 ml flüssigen Ammoniak bei — 60 °C gibt man 11,7 g (300 mmol) gepulvertes Natriumamid und setzt danach langsam eine Mischung aus 29,1 g (300 mmol) n-Capronsäurenitril und 26,4 g (300 mmol) Ethylacetat zu. Man rührt 2 Stunden bei — 60 °C nach, fügt 200 ml Diethylether hinzu und läßt über Nacht auf Raumtemperatur erwärmen, wobei der Ammoniak gasförmig entweicht. Unter Eiskühlung setzt man zuerst Wasser, dann 50 proz. Schwefelsäure zu, bis pH 5 erreicht ist. Die Etherphase trennt man ab, extrahiert die wäßrige Phase zweimal mit Ether, trocknet die vereinigten organischen Phasen und dampft ein. Das zurückbleibende Öl (40 g ; 96 % Rohausbeute) wird im Vakuum destilliert. Die bei 52 °C/0,4 mbar übergehende Fraktion weist eine gaschromatographische Reinheit von über 98 % auf.

b) 9,0 g (65 mmol) Nitril aus Beispiel 2a und 5,5 g (65 mmol)

3-Amino-1,2,4-triazol werden in 200 ml Propionsäure 12 Stunden zum Rückfluß erhitzt. Danach läßt man abkühlen und engt auf ein Drittel des ursprünglichen Lösungsvolumens ein. Nach einiger Zeit bildet sich ein Niederschlag, der abfiltriert, mit wenig 2 N NaOH und Wasser gewaschen und aus Ethanol umkristallisiert wird. Ausbeute 8,6 g (65 %), Schmp. 246 °C (Verbindung Nr. 29).

3. Herstellung von 7-Amino-6-n-butyl-2-methyl-5-n-propyl-pyrazolo-[1,5-a] pyrimidin (Verbindung 20)

a) 2-n-Butyro-n-capronsäure-nitril

Wie in Beispiel 2a angegeben, werden 29,1 g (300 mmol) n-Capronsäure-nitril mit 34,8 g (300 mmol) Ethyl-n-butyrat zur Reaktion gebracht. Rohausbeute 98 %, Reinausbeute 50 %, Sdp. 94 bis 95 °C/0,2 mbar.

b) 10,1 g (60,5 mmol) Nitril aus 3a und 5,80 g (300 mmol)

3(5)-Amino-5(3)-methyl-pyrazol werden wie in Beispiel 2b beschrieben umgesetzt. Ausbeute 5,0 g (34 %), Schmp. (aus Ethanol) 167 °C (Verbindung Nr. 20).

4. Herstellung von 7-Amino-5-methyl-6-[3-(n-nonyl-oxy)-propyl]-[1,2,4]-triazolo [1,5-a]-pyrimidin (Verbindung 39)

a) 2-Acetyl-5-(n-nonyl-oxy)-valeronitril

35,0 g (156 mmol) 5-(n-Nonyl-oxy)-valeronitril werden unter Wasserausschluß und in Inertgasatmosphäre in 500 ml absolutem Tetrahydrofuran vorgelegt, auf — 60 °C abgekühlt, langsam unter Rühren mit 162 mmol n-Butyllithium in n-Hexan versetzt und anschließend 4 Stunden bei — 60 °C belassen. Danach tropft man 13,1 g (155 mmol) trockenes Ethylacetat, gelöst in Tetrahydrofuran, zu, rührt 3 Stunden bei — 60 °C nach und läßt langsam auf Raumtemperatur erwärmen. Unter Eiskühlung wird zunächst Wasser, dann 2 N Salzsäure zugegeben, bis die Lösung pH 4 aufweist. Die sich abscheidende organische Phase wird abgetrennt, mit Wasser gewaschen, getrocknet und eingeengt. Das zurückbleibende Öl (41 g ; 99 %) kann ohne weitere Reinigung umgesetzt werden (gaschromatographische Reinheit > 90 %).

b) 20,0 g (75 mmol) Nitril aus Beispiel 4a werden mit 6,30 g

(75 mmol) 3-Amino-1,2,4-triazol in 300 ml siedender Propionsäure 16 Stunden zur Reaktion gebracht. Man kühlt ab, trennt vom ausgefallenen Niederschlag ab, engt das Filtrat ein, nimmt in Wasser auf, extrahiert mehrmals mit Dichlormethan, trocknet die vereinigten organischen Phasen und dampft ein. Der ölige Rückstand ergibt nach Anreiben mit Diethylether 9,0 (36 %) kristalline Verbindung Nr. 39 vom Schmp. 167 °C.

5. Herstellung von 7-Amino-5-methyl-6-[3-(neopentyl-oxy)-propyl]-[1,2,4]-triazolo [1,5-a]-pyrimidin (Verbindung 33)

a) 7-Hydroxy-5-methyl-6-(3-(neopentyl-oxy)-propyl]-[1,2,4]-triazolo [1,5-a]-pyrimidin

22,0 g (90 mmol) 2-[3-(Neopentyl-oxy)-propyl]-acetessigsäuremethylester und 7,14 g (84,9 mmol) 3-Amino-1,2,4-triazol läßt man 21 Stunden in 300 ml siedender Propionsäure reagieren. Nach Abkühlen wird eingeengt, in Eiswasser eingerührt, mit 2 N NaOH neutralisiert und abgesaugt. Ausbeute 13,5 g (57 %) farblose Kristalle mit Schmp. 155 bis 159 °C, die ohne weitere Reinigung umgesetzt werden.

b) 7-Chlor-5-methyl-6-[3-(neopentyl-oxy)-propyl]-[1,2,4]-triazolo [1,5-a]-pyrimidin

11,5 g (41,4 mmol) Produkt aus 5a erhitzt man 8 Stunden in 330 ml Phosphoroxytrichlorid zum Rückfluß. Nach Abdestillieren flüchtiger Bestandteile bleibt ein öliger Rückstand, der in Dichlormethan aufgenommen, mit gesättigter Natriumhydrogencarbonatlösung gewaschen, getrocknet und eingeengt wird. Es verbleiben 10 g (82 %) eines gelblichen Öls,das dünnschichtchromatographisch rein ist.

c) 10,0 g (33,7 mmol) Produkt aus 5b werden in 100 ml Dioxan gelöst und im Autoklaven mit 6,3 g (370 mmol) Ammoniak 60 Stunden bei 130 °C zur Reaktion gebracht. Nach Abkühlen und Entspannen wird der entstandene Niederschlag abgesaugt, in Dichlormethan aufgenommen, dreimal mit Wasser gewaschen, getrocknet und eingeengt. Man erhält ein farbloses Pulver (Verbindung Nr. 33), Ausbeute 5,5 g (58 %), Schmp. 202 °C. Durch Einengen des Dioxan-Filtrats lassen sich weitere 2,5 g Produkt gewinnen.

Nach den oben beschriebenen Verfahren können z. B. folgende 7-Amino-azolo [1,5-a]-pyrimidine hergestellt werden :

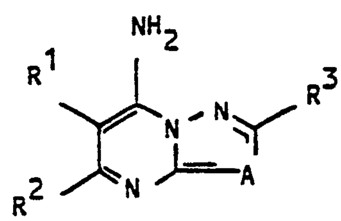

(I)

| Nr. | $R^1$ | $R^2$ | $R^3$ | $R^4$ | A | Fp (°C) |
|---|---|---|---|---|---|---|
| 1 | $n-C_4H_9$ | $CH_3$ | $CH_3$ | H | $CR^4$ | 186 |
| 2 | $n-C_8H_{17}$ | $CH_3$ | $CH_3$ | H | $CR^4$ | 169 |
| 3 | $n-C_4H_9-CH(C_2H_5)-CH_2-$ | $CH_3$ | $CH_3$ | H | $CR^4$ | 159 |
| 4 | $n-C_8H_{17}$ | $CH_3$ | H | - | N | 217 |
| 5 | $n-C_4H_9-CH(C_2H_5)-CH_2-$ | $CH_3$ | H | - | N | 228 |

(Fortsetzung)

| Nr. | $R^1$ | $R^2$ | $R^3$ | $R^4$ | A | Fp (°C) |
|---|---|---|---|---|---|---|
| 6 | $n-C_5H_{11}$ | $CH_3$ | H | – | N | 251 |
| 7 (bekannt). | $n-C_6H_{13}$ | $CH_3$ | H | – | N | 237 |
| 8 | $n-C_7H_{15}$ | $CH_3$ | H | – | N | 209 |
| 9 | $n-C_5H_{11}$ | $CH_3$ | $CH_3$ | – | N | 193 |
| 10 | $n-C_6H_{13}$ | $CH_3$ | $CH_3$ | – | N | 200 |
| 11 | $n-C_8H_{17}$ | $CH_3$ | $CH_3$ | – | N | 198 |
| 12 | $n-C_4H_9$ | $n-C_4H_9$ | H | – | N | 187 |
| 13 | $n-C_4H_9$ | $n-C_4H_9$ | $CH_3$ | H | $CR^4$ | 145 |
| 14 | $n-C_3H_7-CH(CH_3)-CH_2-$ | $CH_3$ | H | – | N | 209 |
| 15 | $n-C_3H_7-CH(CH_3)-CH_2-$ | $CH_3$ | $CH_3$ | H | $CR^4$ | 176 |
| 16 | $(H_5C_2)_2-CH-CH_2-$ | $CH_3$ | $CH_3$ | H | $CR^4$ | 178 |
| 17 | $n-C_{10}H_{21}$ | $n-C_3H_7$ | H | – | N | 152 |
| 18 | $n-C_{10}H_{21}$ | $n-C_3H_7$ | $CH_3$ | H | $CR^4$ | 114 |
| 19 | $n-C_{10}H_{21}$ | $n-C_4H_9$ | H | – | N | 151 |
| 20 | $n-C_5H_9$ | $n-C_3H_7$ | $CH_3$ | H | $CR^4$ | 167 |
| 21 | $n-C_4H_9$ | $n-C_3H_7$ | H | – | N | 186 |
| 22 | $n-C_{12}H_{25}$ | $CH_3$ | $CH_3$ | – | $CR^4$ | 127 |
| 23 | $n-C_{12}H_{25}$ | $CH_3$ | H | – | N | 209 |
| 24 | $n-C_{10}H_{21}$ | $CH_3$ | H | – | N | 207 |
| 25 | $n-C_9H_{19}$ | $CH_3$ | H | – | N | 215 |
| 26 | $n-C_6H_{13}$ | $CH_3$ | $CH_3$ | H | $CR^4$ | 177 |
| 27 | $n-C_9H_{19}$ | $CH_3$ | $CH_3$ | – | N | 159 |
| 28 | $n-C_{10}H_{21}$ | $CH_3$ | $CH_3$ | H | $CR^4$ | 146 |
| 29 | $n-C_4H_9$ | $CH_3$ | H | – | N | 246 |
| 30 | $(n-C_3H_7)(C_2H_5)CHCH_2-O-(CH_2)_3-$ | $CH_3$ | $CH_3$ | H | $CR^4$ | 108 |
| 31 | $(n-C_3H_7)(C_2H_5)CHCH_2-O-(CH_2)_3-$ | $CH_3$ | H | – | N | 149 |
| 32 | $t-C_4H_9CH_2-O-(CH_2)_3-$ | $CH_3$ | $CH_3$ | H | $CR^4$ | 134 |
| 33 | $t-C_4H_9-CH_2-O-(CH_2)_3-$ | $CH_3$ | H | – | N | 202 |
| 34 | $t-C_4H_9-O-(CH_2)_4-$ | $CH_3$ | $CH_3$ | H | $CR^4$ | 61 (Zers.) |
| 35 | $(t-C_4H_9-CH_2)(CH_3)CH(CH_2)_2-O-(CH_2)_5-$ | $CH_3$ | H | – | N | 161 |
| 36 | $(i-C_4H_9)(C_2H_5)CH-O-(CH_2)_3-$ | $CH_3$ | H | – | N | 156 |
| 37 | $n-C_8H_{17}-O-(CH_2)_3-$ | $n-C_3H_7$ | H | – | N | 97 |
| 38 | $i-C_3H_7-(CH_2)_3-CH(CH_3)-(CH_2)_2-O-(CH_2)_3-$ | $CH_3$ | H | – | N | 156 |
| 39 | $n-C_9H_{19}-O-(CH_2)_3-$ | $CH_3$ | H | – | N | 167 |
| 40 | $n-C_4H_9-O-(CH_2)_3-$ | $CH_3$ | H | – | N | 180 |
| 41 | $n-C_6H_{13}-O-(CH_2)_3-$ | $CH_3$ | H | – | N | 176 |

(Fortsetzung)

| Nr. | $R^1$ | $R^2$ | $R^3$ | $R^4$ | A | Fp (°C) |
|---|---|---|---|---|---|---|
| 42 | $n-C_9H_{19}-O-(CH_2)_3-$ | $CH_3$ | $CH_3$ | H | $CR^4$ | 94 |
| 43 | $n-C_6H_{13}-O-(CH_2)_3-$ | $n-C_3H_7$ | H | - | N | 109 |
| 44 | $(n-C_4H_9)(C_2H_5)CH-CH_2-O-(CH_2)_5-$ | $CH_3$ | H | - | N | Harz |
| 45 | $t-C_4H_9-CH_2-CH(CH_3)-(CH_2)_2-$ $-O-(CH_2)_3-$ | $CH_3$ | H | - | N | 154 |
| 46 | $n-C_4H_9-O-(CH_2)_3-$ | $CH_3$ | $CH_3$ | H | $CR^4$ | 145 |
| 47 | $t-C_4H_9-CH_2-CH(CH_3)-(CH_2)_2-$ $-O-(CH_2)_3-$ | $CH_3$ | $CH_3$ | H | $CR^4$ | 122 |

Die Wirkstoffe der Formel I zeigen eine starke fungitoxische Wirksamkeit gegen phytopathogene Pilze, insbesondere aus der Klasse der Phycomyceten. Die Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an Sonnenblumen, Sclerospora macrospora an Mais, Bremia lactucae an Salat, Mucor mucedo an Früchten, Rhizopus nigricans an Rüben sowie von Erysiphe graminis an Getreide, Uncinula necator an Reben, Podophaera leucotricha an Äpfeln, Sphaerotheca fuliginea an Rosen, Erysiphe cichoriacearum an Gurken.

Die Wirkstoffe besitzen eine hohe Pflanzenverträglichkeit. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 bis 5 kg Wirkstoff je ha.

Die Wirkstoffe können auch zusammen mit anderen Wirkstoffen, z. B. Herbiziden, Insektiziden, Wachstumsregulatoren und anderen Fungiziden, oder auch mit Düngemitteln vermischt ausgebracht werden. In vielen Fällen erhält man bei der Mischung mit Fungiziden auch eine Vergrößerung des fungiziden Wirkungsspektrums ; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten.

Fungizide, die mit den erfindungsgemäßen Verbindungen kombiniert werden können, sind beispielsweise :

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
2-Cyano-N-(ethylaminocarbonyl)-2-(methoximino)-acetamid

sowie verschiedene Fungizide, wie
Dodecylguanidinacetat
3-(3-(3,5-Dimethyl-2-oxycyclohexyl)-2-hydroxyethyl)-glutaramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.

Die erfindungsgemäßen Wirkstoffe werden beispielsweise in Form von direkt versprühbaren Lösungen, Suspensionen, Emulsionen, auch in Form von hochprozentigen wäßrigen, öligen oder

sonstigen Dispersionen, Pasten, Stäubemitteln, Streumitteln, Granulaten durch Versprühen, Verstäuben, Verstreuen, Verstreichen oder Gießen ausgebracht. Die Anwendungsformen richten sich ganz nach den Verwendungszwecken ; sie sollten in der Regel möglichst eine feine Verteilung der neuen Wirkstoffe gewährleisten.

Zur Herstellung von direkt oder nach Emulgieren in Wasser verwendbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische oder aromatische Kohlenwasserstoffe, zum Beispiel Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumlsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Pflanzenschutzmittel-Zubereitungen sind :

I. Man vermischt 90 Gewichtsteile der Verbindung des Beispiels 1 mit 100 Gewichtsteilen N-Methylpyrrolidon und erhält eine Lösung, die zur Anwendung in Form kleinster Tropfen geeignet ist.

II. 20 Gewichtsteile der Verbindung des Beispiels 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Doedecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der nach Beispiel 1 erhältlichen Verbindung werden in einer Mischung gelöst, die aus 30 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und Verrühren der Lösung in Wasser erhält man eine wäßrige Dispersion.

IV. 20 Gewichtsteile der nach Beispiel 2 erhältlichen Verbindung werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und Verrühren der Lösung in Wasser, erhält man eine wäßrige Dispersion.

V. 20 Gewichtsteile der nach Beispiel 1 erhältlichen Verbindung werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in Wasser erhält man eine Spritzbrühe.

VI. 5 Gewichtsteile der nach Beispiel 2 erhältlichen Verbindung werden mit 95 Gewichtsteilen feinteiligem Kaolin innig vermischt. Man erhält auf diese Weise ein Stäubemittel, das 5 Gew.-% des Wirkstoffs enthält.

VII. 30 Gewichtsteile der nach Beispiel 1 erhältlichen Verbindung werden mit einer Mischung aus 92

Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der nach Beispiel 2 erhältlichen Verbindung werden mit 30 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion.

IX. 20 Teile der nach Beispiel 1 erhältlichen Verbindung werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die folgenden Versuche belegen die biologische Wirkung der erfindungsgemäßen Verbindungen.

Versuch 1
Wirksamkeit gegen Plasmopara viticola

Blätter von Topfreben der Sorte « Müller-Thurgau » werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24 °C und anschließend für 8 Tage in einem Gewächshaus mit Temperaturen zwischen 20 und 30 °C aufgestellt. Nach dieser Zeit werden die Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß beispielsweise bei der Anwendung als 0,05 oder 0,025 %ige Wirkstoffbrühe die Wirkstoffe 1, 2, 3, 4, 5, 6, 7, 8, 9, 10, 11, 12, 13, 14, 15, 16, 17, 18, 21, 22, 24, 25, 26, 27 und 28 eine bessere fungizide Wirkung (beispielsweise 97 %) zeigen als die bekannten Wirkstoffe 7-Amino-6-phenyl-(1,2,4-triazolo)-[1,5-a]-pyrimidin (A) und 7-Amino-2-methyl-6-phenyl-pyrazolo-[1,5-a]-pyrimidin (B) (beispielsweise 30 %).

Versuch 2
Wirksamkeit gegen Phytophthora infestans an Tomaten

Blätter von Topfpflanzen der Sorte « Große Fleischtomate » werden mit wäßriger Spritzbrühe, die 80 % Wirkstoff und 20 % Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise bei der Anwendung als 0,025 %ige Wirkstoffbrühe die Wirkstoffe 4, 5, 6, 7, 9, 10, 11, 12, 14, 15, 16, 17, 21, 25, 31, 35, 36, 43 und 44 eine bessere fungizide Wirkung zeigten (beispielsweise 90 %) als die bekannten Wirkstoffe A und N-Trichlormethylthio-tetrahydrophthalimid (beispielsweise 60 %).

**Patentansprüche** (für die Vertragsstaaten : BE, CH, DE, FR, GB, IT, LI, NL, SE)

1. 7-Amino-azolo [1,5-a]-pyrimidine der Formel

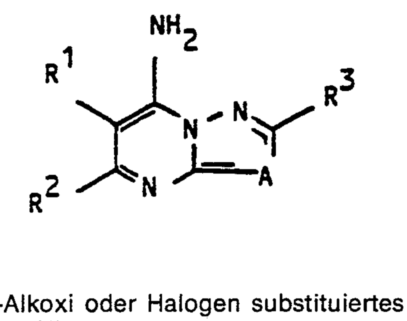

(I)

worin

$R^1$ gegebenenfalls durch $C_1$-$C_{18}$-Alkoxi oder Halogen substituiertes $C_4$-$C_{18}$-Alkyl,

$R^2$ und $R^3$ Wasserstoff oder $C_1$-$C_4$-Alkyl,

A ein Stickstoffatom oder eine $CR^4$-Gruppe bedeutet, wobei $R^4$ die Bedeutung Wasserstoff, $C_1$-$C_4$-Alkyl oder Halogen hat

außer der Verbindung 7-Amino-6-hexyl-5-methyl-[1,2,4]-triazolo [1,5-a]-pyrimidin.

2. Fungizides Mittel, gekennzeichnet durch einen Gehalt an einem 7-Amino-azolo [1,5-a]-pyrimidin der Formel I gemäß Anspruch 1.

3. Fungizides Mittel enthaltend einen festen oder flüssigen Trägerstoff und Aminoazolopyrimidin der Formel I gemäß Anspruch 1.

4. Verfahren zur Bekämpfung von Pilzen, dadurch gekennzeichnet, daß man die Pilze oder die vor Pilzbefall zu schützenden Materialien, Pflanzen, Boden oder Saatgüter behandelt mit einer fungizid wirksamen Menge eines Aminoazolopyrimidins der Formel

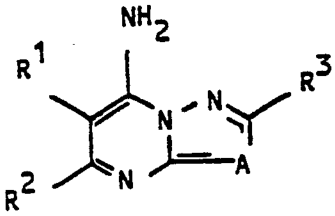

(I)

worin

$R^1$ gegebenenfalls durch $C_1$-$C_{18}$-Alkoxi oder Halogen substituiertes $C_4$-$C_{18}$-Alkyl,

$R^2$ und $R^3$ Wasserstoff oder $C_1$-$C_4$-Alkyl,

A ein Stickstoffatom oder eine $CR^4$-Gruppe bedeutet, wobei $R^4$ die Bedeutung Wasserstoff, $C_1$-$C_4$-Alkyl oder Halogen hat.

5. Verfahren zur Herstellung von 7-Amino-azolo [1,5-a]-pyrimidinen der Formel I gemäß Anspruch 1, dadurch gekennzeichnet, daß man einen substituierten β-Ketoester der Formel II

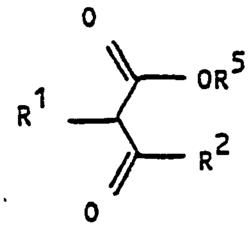

(II)

in der $R^1$ und $R^2$ die oben genannten Bedeutungen haben und $R^5$ für einen niederen Alkylrest steht, mit einem Aminoazol der Formel III

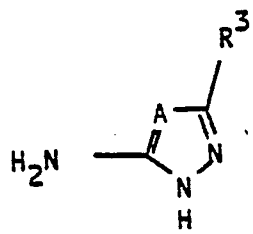

(III)

in welcher A, $R^3$ und $R^4$ die oben angegebenen Bedeutungen haben, zu Kondensationsprodukten der Formel V

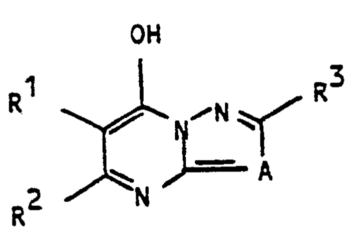

(V)

in welcher $R^1$, $R^2$, $R^3$ und A die im Anspruch 1 angegebenen Bedeutungen haben, umsetzt und diese Kondensationsprodukte der Formel V nach Halogenierung der Hydroxigruppe mit Ammoniak umsetzt.

6. Verfahren zur Herstellung von 7-Amino-azolo [1,5-]-pyrimidinen der Formel I gemäß Anspruch 1, dadurch gekennzeichnet, daß man ein substituiertes Alkylcyanid der Formel

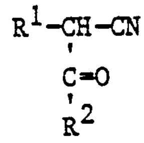

(VI)

in der $R^1$ und $R^2$ die oben genannte Bedeutung haben, mit einem Aminoazol der Formel

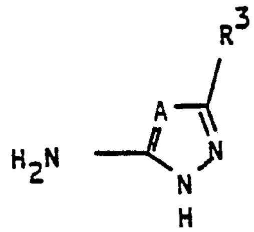

(III)

in welcher A, $R^3$ und $R^4$ die oben angegebenen Bedeutungen haben, umsetzt.

7. Verbindung gemäß Anspruch 1, dadurch gekennzeichnet, daß $R^1$ Heptyl, Octyl, Nonyl, Decyl, Undecyl oder Dodecyl bedeutet.

8. Verbindung gemäß Anspruch 1, dadurch gekennzeichnet, daß $R^1$ Octyl, $R^2$ Methyl, $R^3$ Wasserstoff und A N bedeutet.

**Patentansprüche** (für den Vertragsstaat AT)

1. Fungizides Mittel, gekennzeichnet durch einen Gehalt an einem 7-Amino-azolo [1,5-a]-pyrimidin der Formel

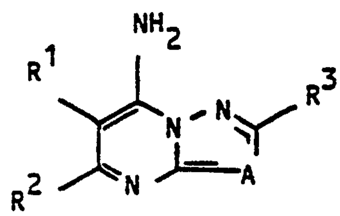

(I)

worin
$R^1$ gegebenenfalls durch $C_1$-$C_{18}$-Alkoxi oder Halogen substituiertes $C_4$-$C_{18}$-Alkyl,
$R^2$ und $R^3$ Wasserstoff oder $C_1$-$C_4$-Alkyl,
A ein Stickstoffatom oder eine $CR^4$-Gruppe bedeutet, wobei $R^4$ die Bedeutung Wasserstoff, $C_1$-$C_4$-Alkyl oder Halogen hat
außer der Verbindung 7-Amino-6-hexyl-5-methyl-[1,2,4]-pyrimidin.

2. Fungizides Mittel enthaltend einen festen oder flüssigen Trägerstoff und Aminoazolopyrimidin der Formel I gemäß Anspruch 1.

3. Verfahren zur Bekämpfung von Pilzen, dadurch gekennzeichnet, daß man die Pilze oder die vor Pilzbefall zu schützenden Materialien, Pflanzen, Boden oder Saatgüter behandelt mit einer fungizid wirksamen Menge eines Aminoazolopyrimidins der Formel

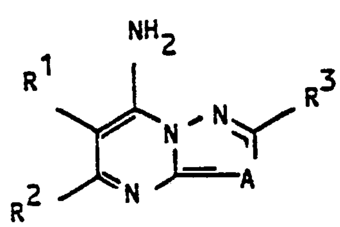

(I)

worin
$R^1$ gegebenenfalls durch $C_1$-$C_{18}$-Alkoxi oder Halogen substituiertes $C_4$-$C_{18}$-Alkyl,
$R^2$ und $R^3$ Wasserstoff oder $C_1$-$C_4$-Alkyl,
A ein Stickstoffatom oder eine $CR^4$-Gruppe bedeutet, wobei $R^4$ die Bedeutung Wasserstoff, $C_1$-$C_4$-Alkyl oder Halogen hat.

4. Verfahren zur Herstellung von 7-Amino-azolo [1,5-a]-pyrimidinen der Formel I gemäß Anspruch 1, dadurch gekennzeichnet, daß man einen substituierten β-Ketoester der Formel II

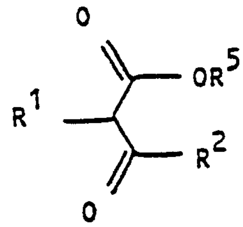

(II)

in der $R^1$ und $R^2$ die oben genannten Bedeutungen haben und $R^5$ für einen niederen Alkylrest steht, mit einem Aminoazol der Formel III

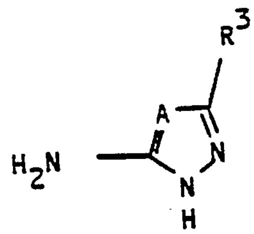

(III)

in welcher A, $R^3$ und $R^4$ die oben angegebenen Bedeutungen haben, zu Kondensationsprodukten der Formel V

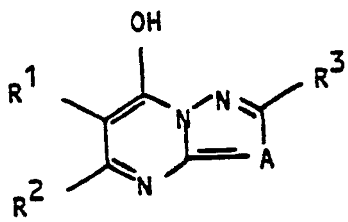

(V)

in welcher $R^1$, $R^2$, $R^3$ und A die im Anspruch 1 angegebenen Bedeutungen haben, umsetzt und diese Kondensationsprodukte der Formel V nach Halogenierung der Hydroxigruppe mit Ammoniak umsetzt.

5. Verfahren zur Herstellung von 7-Amino-azolo [1,5-a]-pyrimidinen der Formel I gemäß Anspruch 1, dadurch gekennzeichnet, daß man ein substituiertes Alkylcyanid der Formel

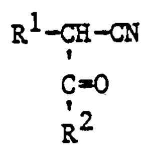

(VI)

in der $R^1$ und $R^2$ die oben genannte Bedeutung haben, mit einem Aminoazol der Formel

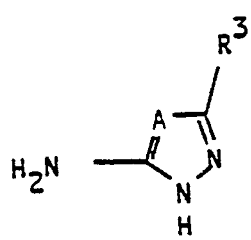

(III)

in welcher A, $R^3$ und $R^4$ die oben angegebenen Bedeutungen haben, umsetzt.

6. Fungizides Mittel gemäß Anspruch 2, dadurch gekennzeichnet, daß $R^1$ Heptyl, Octyl, Nonyl, Decyl, Undecyl oder Dodecyl bedeutet.

7. Fungizides Mittel gemäß Anspruch 2, dadurch gekennzeichnet, daß $R^1$ Octyl, $R^2$ Methyl, $R^3$ Wasserstoff und A N bedeutet.

**Claims** (for the Contracting States : BE, CH, DE, FR, GB, IT, LI, NL, SE)

1. A 7-aminoazolo [1,5-a] pyrimidine of the formula

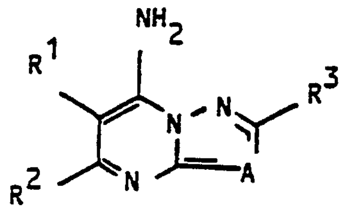

(I)

where
$R^1$ is $C_4$-$C_{18}$-alkyl which is unsubstituted or substituted by $C_1$-$C_{18}$-alkoxy or halogen,
$R^2$ and $R^3$ are each hydrogen or $C_1$-$C_4$-alkyl and
A is a nitrogen atom or a $CR^4$ group, where $R^4$ is hydrogen, $C_1$-$C_4$-alkyl or halogen,
with the exception of the compound 7-amino-6-hexyl-5-methyl-[1.2.4] triazolo [1,5-a] pyrimidine.

2. A fungicide which contains a 7-aminoazolo [1,5-a] pyrimidine of the formula I a claimed in claim 1.

3. A fungicide which contains a solid or liquid carrier and an aminoazolopyrimidine of the formula I as claimed in claim 1.

4. A method of controlling fungi, wherein the fungi or the materials, plants, soil or seed to be protected from fungal attack are treated with an fungicidal amount of an aminoazolopyrimidine of the formula

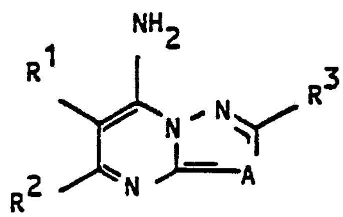

(I)

where
$R^1$ is $C_4$-$C_{18}$-alkyl which is unsubstituted or substituted by $C_1$-$C_{18}$-alkoxy or halogen,
$R^2$ and $R^3$ are each hydrogen or $C_1$-$C_4$-alkyl and
A is a nitrogen atom or a $CR^4$ group, where $R^4$ is hydrogen, $C_1$-$C_4$-alkyl or halogen.

5. A process for the preparation of a 7-aminoazolo-[1,5-a] pyrimidine of the formula I as claimed in claim 1, wherein a substituted β-ketoester of the formula II

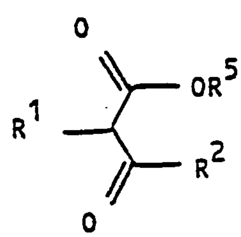

(II)

where $R^1$ and $R^2$ have the above meanings and $R^5$ is a lower alkyl radical, is reacted with an aminoazole of the formula III

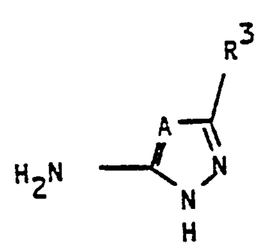

(III)

where A, $R^3$ and $R^4$ have the above meanings, to give a condensate of the formula V

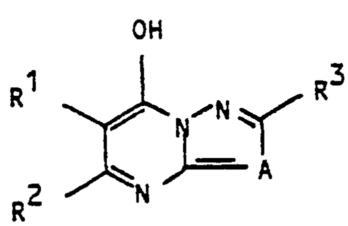

(V)

where $R^1$, $R^2$, $R^3$ and A have the meanings given in claim 1, and this condensate of the formula V is reacted with ammonia after halogenation of the hydroxyl group.

6. A process for the preparation of a 7-aminoazolo-[1,5-a] pyrimidine of the formula I as claimed in claim 1, wherein a substituted alkyl cyanide of the formula

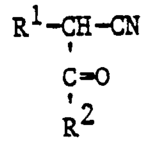

(VI)

where $R^1$ and $R^2$ have the above meanings, is reacted with an aminoazole of the formula

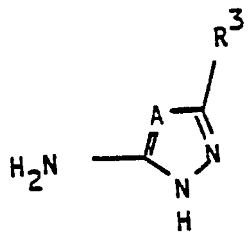

(III)

where A, $R^3$ and $R^4$ have the above meanings.

7. A compound as claimed in claim 1, wherein $R^1$ is heptyl, octyl, nonyl, decyl, undecyl or dodecyl.

8. A compound as claimed in claim 1, wherein $R^1$ is octyl, $R^2$ is methyl, $R^3$ is hydrogen and A is N.

**Claims** (for the Contracting State AT)

1. A fungicide which contains a 7-aminoazolo [1,5-a] pyrimidine of the formula

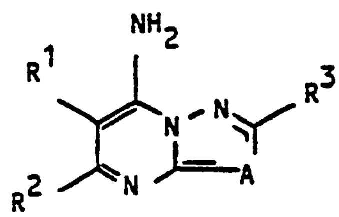

(I)

where

$R^1$ is $C_4$-$C_{18}$-alkyl which is unsubstituted or substituted by $C_1$-$C_{18}$-alkoxy or halogen,

$R^2$ and $R^3$ are each hydrogen or $C_1$-$C_4$-alkyl and

A is a nitrogen atom or a $CR^4$ group, where $R^4$ is hydrogen, $C_1$-$C_4$-alkyl or halogen,

with the exception of the compound 7-amino-6-hexyl-5-methyl-[1.2.4] triazolo [1,5-a] pyrimidine.

2. A fungicide which contains a solid or liquid carrier and an aminoazolopyrimidine of the formula I as claimed in claim 1.

3. A method of controlling fungi, wherein the fungi or the materials, plants, soil or seed to be protected from fungal attack are treated with a fungicidal amount of an aminoazolopyrimidine of the formula

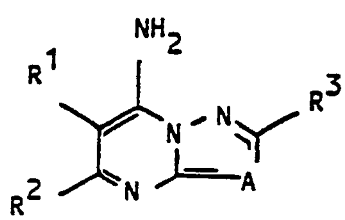

(I)

where

$R^1$ is $C_4$-$C_{18}$-alkyl which is unsubstituted or substituted by $C_1$-$C_{18}$-alkoxy or halogen,

$R^2$ and $R^3$ are each hydrogen or $C_1$-$C_4$-alkyl and

A is a nitrogen atom or a $CR^4$ group, where $R^4$ is hydrogen, $C_1$-$C_4$-alkyl or halogen.

4. A process for the preparation of a 7-aminoazolo-[1,5-a] pyrimidine of the formula I as claimed in claim 1, wherein a substituted β-ketoester of the formula II

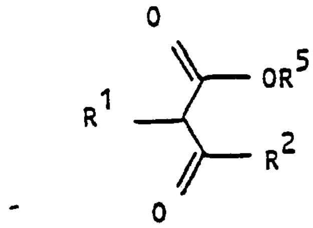

(II)

where $R^1$ and $R^2$ have the above meanings and $R^5$ is a lower alkyl radical, is reacted with an aminoazole of the formula III

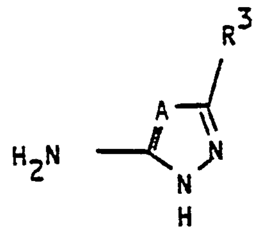

(III)

where A, $R^3$ and $R^4$ have the above meanings, to give a condensate of the formula V

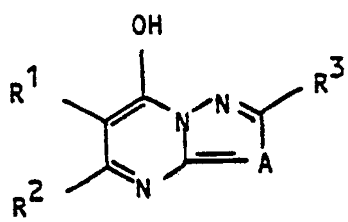

(V)

where $R^1$, $R^2$, $R^3$ and A have the meanings given in claim 1, and this condensate of the formula V is reacted with ammonia after halogenation of the hydroxyl group.

5. A process for the preparation of a 7-aminoazolo-[1,5-a] pyrimidine of the formula I as claimed in claim 1, wherein a substituted alkyl cyanide of the formula

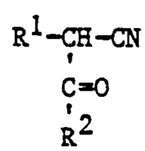

(VI)

where $R^1$ and $R^2$ have the above meanings, is reacted with an aminoazole of the formula

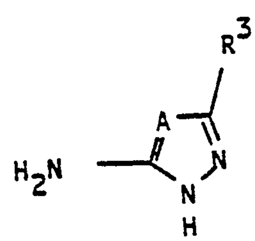

(III)

where A, $R^3$ and $R^4$ have the above meanings.

6. A fungicide as claimed in claim 2, wherein $R^1$ is heptyl, octyl, nonyl, decyl, undecyl or dodecyl.

7. A fungicide as claimed in claim 2, wherein $R^1$ is octyl, $R^2$ is methyl, $R^3$ is hydrogen and A is N.

**Revendications** (pour les Etats contractants : BE, CH, DE, FR, GB, IT, LI, NL, SE)

1. 7-Amino-azolo [1,5-a]-pyrimidines de la formule

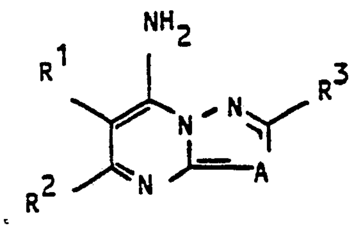

(I)

dans laquelle

$R^1$ représente un radical alkyle en $C_4$ à $C_{18}$ éventuellement alcoxy (en $C_1$ à $C_{18}$)- ou halo-substitué,

$R^2$ et $R^3$ désignent chacun un atome d'hydrogène ou un radical alkyle en $C_1$ à $C_4$ et

A désigne un atome d'azote ou un groupe —$CR^4$, dans lequel $R^4$ représente un atome d'hydrogène ou d'halogène ou un radical alkyle en $C_1$ à $C_4$,

à l'exception de la 7-amino-6-hexyl-5-méthyl-[1,2,4]-triazolo [1,5-a]-pyrimidine.

2. Composition fongicide, caractérisée en ce qu'elle contient une 7-amino-azolo [1,5-a]-pyrimidine de la formule I selon la revendication 1.

3. Composition fongicide, contenant une amino-azolo-pyrimidine de la formule I selon la revendication 1 dans un véhicule solide ou liquide.

4. Procédé de lutte contre des champignons, caractérisé en ce que l'on traite les champignons ou les matériaux, sols, plantes ou semences à protéger des champignons avec une quantité efficace du point de vue fongicide d'une amino-azolo-pyrimidine de la formule

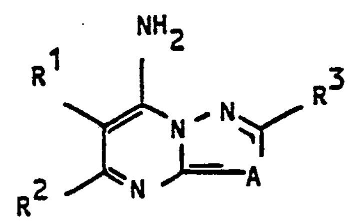

(I)

dans laquelle

$R^1$ représente un radical alkyle en $C_4$ à $C_{18}$ éventuellement alcoxy (en $C_1$ à $C_{18}$)- ou halo-substitué,

$R^2$ et $R^3$ désignent chacun un atome d'hydrogène ou un radical alkyle en $C_1$ à $C_4$ et

A désigne un atome d'azote ou un groupe —$CR^4$, dans lequel $R^4$ représente un atome d'hydrogène ou d'halogène ou un radical alkyle en $C_1$ à $C_4$.

5. Procédé de préparation de 7-amino-azolo [1,5-a]-pyrimidines de la formule I selon la revendication 1, caractérisé en ce que, à partir d'un β-céto-ester substitué de la formule II

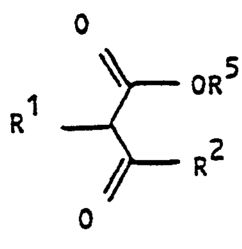

(II)

dans laquelle $R^1$ et $R^2$ possèdent les significations définies et $R^5$ désigne un radical alkyle inférieur, et d'un amino-azole de la formule III

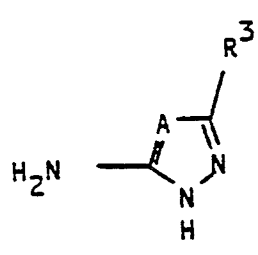

(III)

dans laquelle $R^3$, $R^4$ et A possèdent les significations définies plus haut, on prépare des produits de condensation de la formule V

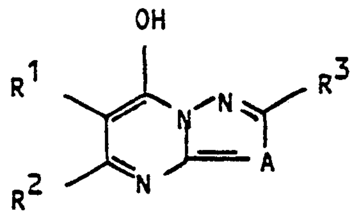

(V)

dans laquelle $R^1$, $R^2$, $R^3$ et A possèdent les significations définies dans la revendication 1, et après l'halogénation du groupe oxhydryle, on fait réagir les produits de condensation de la formule V avec de l'ammoniac.

6. Procédé de préparation de 7-amino-azolo-[1,5-a]-pyrimidines de la formule I selon la revendication 1, caractérisé en ce que l'on fait réagir un cyanure d'alkyle substitué de la formule

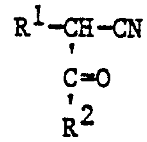

(VI)

dans laquelle $R^1$ et $R^2$ possèdent la signification définie, avec un amino-azole de la formule

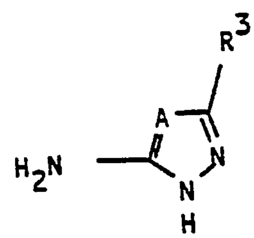

(III)

dans laquelle $R^3$, $R^4$ et A possèdent les significations définies plus haut.

7. Composé suivant la revendication 1, caractérisé en ce que $R^1$ désigne un groupe heptyle, octyle, nonyle, décyle, undécyle ou dodécyle.

8. Composé suivant la revendication 1, caractérisé en ce que $R^1$ désigne un groupe octyle, $R^2$ un radical méthyle, $R^3$ un atome d'hydrogène et A un atome d'azote.

**Revendications** (pour l'Etat contractant AT)

1. Composition fongicide, caractérisée en ce qu'elle contient une 7-amino-azolo [1,5-a]-pyrimidine de la formule

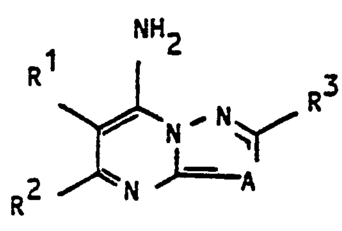

(I)

dans laquelle

$R^1$ représente un radical alkyle en $C_4$ à $C_{18}$ éventuellement alcoxy (en $C_1$ à $C_{18}$)- ou halo-substitué,

$R^2$ et $R^3$ désignent chacun un atome d'hydrogène ou un radical alkyle en $C_1$ à $C_4$ et

A désigne un atome d'azote ou un groupe —$CR^4$, dans lequel $R^4$ représente un atome d'hydrogène ou d'halogène ou un radical alkyle en $C_1$ à $C_4$,

à l'exception de la 7-amino-6-hexyl-5-méthyl-[1,2,4]-triazolo [1,5-a]-pyrimidine.

2. Composition fongicide, contenant une amino-azolo-pyrimidine de la formule I selon la revendication 1 dans un véhicule solide ou liquide.

3. Procédé de lutte contre des champignons, caractérisé en ce que l'on traite les champignons ou les matériaux, sols, plantes ou semences à protéger des champignons avec une quantité efficace du point de vue fongicide d'une amino-azolo-pyrimidine de la formule

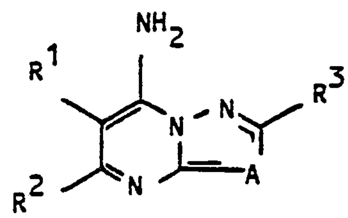

(I)

dans laquelle

$R^1$ représente un radical alkyle en $C_4$ à $C_{18}$ éventuellement alcoxy (en $C_1$ à $C_{18}$)- ou halo-substitué,

$R^2$ et $R^3$ désignent chacun un atome d'hydrogène ou un radical alkyle en $C_1$ à $C_4$ et

A désigne un atome d'azote ou un groupe —$CR^4$, dans lequel $R^4$ représente un atome d'hydrogène ou d'halogène ou un radical alkyle en $C_1$ à $C_4$.

4. Procédé de préparation de 7-amino-azolo-[1,5-a]-pyrimidines de la formule I selon la revendication 1, caractérisé en ce que, à partir d'un β-céto-ester substitué de la formule II

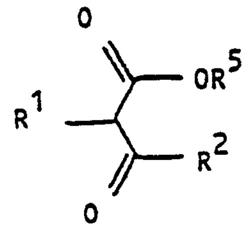

(II)

dans laquelle $R^1$ et $R^2$ possèdent les significations définies et $R^5$ désigne un radical alkyle inférieur, et d'un amino-azole de la formule III

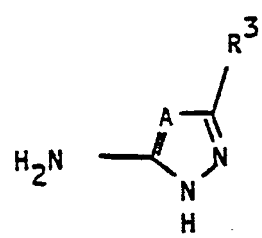

(III)

dans laquelle $R^3$, $R^4$ et A possèdent les significations définies plus haut, on prépare des produits de condensation de la formule V

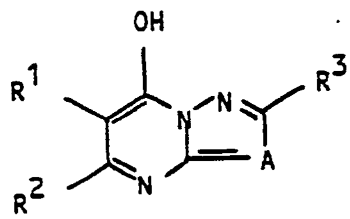

(V)

dans laquelle $R^1$, $R^2$, $R^3$ et A possèdent les significations définies dans la revendication 1, et après l'halogénation du groupe oxhydryle, on fait réagir les produits de condensation de la formule V avec de l'ammoniac.

5. Procédé de préparation de 7-amino-azolo-[1,5-a]-pyrimidines de la formule I selon la revendication 1, caractérisé en ce que l'on fait réagir un cyanure d'alkyle substitué de la formule

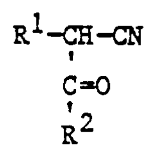

(VI)

dans laquelle $R^1$ et $R^2$ possèdent la signification définie, avec un amino-azole de la formule

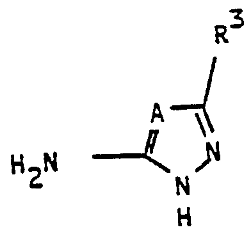

(III)

dans laquelle $R^3$, $R^4$ et A possèdent les significations définies plus haut.

6. Composition fongicide suivant la revendication 2, caractérisée en ce que $R^1$ désigne un groupe heptyle, octyle, nonyle, décyle, undécyle ou dodécyle.

7. Composition fongicide suivant la revendication 2, caractérisée en ce que $R^1$ désigne un groupe octyle, $R^2$ un radical méthyle, $R^3$ un atome d'hydrogène et A un atome d'azote.